(19) Europäisches Patentamt
European Patent Office
Office européen des brevets

(11) **EP 3 886 601 B1**

(12) **EUROPEAN PATENT SPECIFICATION**

(45) Date of publication and mention
of the grant of the patent:
**19.10.2022 Bulletin 2022/42**

(21) Application number: **20746859.6**

(22) Date of filing: **11.06.2020**

(51) International Patent Classification (IPC):
**A23L 3/32** *(2006.01)*

(52) Cooperative Patent Classification (CPC):
(C-Sets available)
**A23B 7/015; A23L 3/32;** A23V 2002/00;
A47J 27/004; A61N 1/327 (Cont.)

(86) International application number:
**PCT/CZ2020/050044**

(87) International publication number:
**WO 2020/249144 (17.12.2020 Gazette 2020/51)**

(54) **DEVICE AND METHOD FOR TREATMENT OF FOOD BY PULSED ELECTRIC FIELD**

VORRICHTUNG UND METHODE ZUR BEHANDLUNG VON LEBENSMITTELN DURCH GEPULSTES ELEKTRISCHES FELD

APPAREIL ET METHODE POUR LE TRAITEMENT D'ALIMENTS PAR CHAMPS ELECTRIQUE PULSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.06.2019 CZ 201936294 U
12.06.2019 CZ 20190366**

(43) Date of publication of application:
**06.10.2021 Bulletin 2021/40**

(73) Proprietor: **Ceská zemedelská univerzita v Praze
165 00 Praha 6 (CZ)**

(72) Inventors:
 • **BLAHOVEC, Jiri
  14900 Praha 4 (CZ)**
 • **KUMHALA, Frantisek
  56914 Vendoli (CZ)**
 • **LEV, Jakub
  27379 Libovice (CZ)**
 • **KOURIM, Pavel
  17000 Praha 7 (CZ)**
 • **JOST, Bohuslav
  16500 Praha 6 (CZ)**

(74) Representative: **Hartvichova, Katerina
HARBER IP s.r.o.
Dukelskych hrdinu 567/52
170 00 Praha 7 (CZ)**

(56) References cited:
EP-A1- 3 503 678      WO-A1-2006/121397
US-A- 4 838 154      US-B2- 6 746 613
US-B2- 9 565 869      US-B2- 10 085 461

(52) Cooperative Patent Classification (CPC): (Cont.)

C-Sets
A23V 2002/00, A23V 2200/26, A23V 2300/12

## Description

### Field of Art

The invention relates to a device for treatment of food (processing of food), in particular fruits and vegetables, for direct consumption or culinary processing. The device works on the principle of pulsed electric field used to damage cell membranes, and thus to release a major part of cell fluids, thereby changing the texture and digestibility of the food without deactivating biologically active substances.

### Background Art

One possible food preparation method is the use of pulsed electric heating at lower temperatures (temperatures are below 42 °C, so such food treatment is also suitable for people following the so-called raw food diet). In this way, the activity of all health-promoting substances can be maintained. Furthermore, the energy requirements of the whole food-processing process may be lower than the energy needed for conventional processing.

The principle of pulsed electric heating or processing is known, and currently it is the subject of very intensive basic and applied research. In recent years, the principle has found use in the food processing industry. The pulsed electric field can be used, for example, to deactivate biological contamination of fluids, as proposed, for example, in U.S. Pat. No. 6,746,613 B2. A similar use of a pulsed electric field is also described in Nestlé's EP 2 543 254 A1, which uses the pulsed electric field to inactivate bioactive components in milk during pasteurization for preservation.

However, systems are also known for applying a pulsed electric field to more or less solid food products, such as meat or vegetables, including potatoes. Examples are disclosed in WO 2006/121397 and WO 2011/139144 A1, which describe methods and systems for such processing.

Currently, around 10 companies worldwide manufacture devices enabling pulsed electric field applications in the food processing industry. However, culinary and kitchen applications in general are still unique and not accessible to a common consumer. The Dutch company IXL Netherlands B.V. The Netherlands (www.innovation-xl.com) produces devices for kitchen applications, but her low-voltage products lack the ability to directly damage cell membranes by electric field. This principle is protected, for example, by U.S. Pat. No. 9,565,869 B2. High-voltage applications are still hindered by performance and safety constraints in households that prevent their wider expansion to small consumers.

Thus, there is still a need to develop a device that can be used in a normal household kitchen-scale processing.

### Summary of the Invention

The present invention achieves the above-mentioned aim by providing a food treatment device (food processing device) comprising an AC voltage source with at least two contact electrodes, wherein each contact electrode is divided into a plurality of segments, and wherein the device further comprises a control unit for controlling the AC voltage source and controlling the voltage supply to the individual electrode segments.

The most important technical feature of this device is the division of each contact electrode into several segments. This allows to distribute the required power input more evenly over time and to automatically regulate the efficiency of the pulsation process in smaller volumes of material to be processed. Thanks to the segmentation of the electrodes, the food to be treated can be subjected to a pulsed electric field gradually, thus achieving sufficient food processing can be achieved with a substantially lower magnitude of the electric current used.

The electrical conductivity G (S) of the material (food) is calculated according to the formula:

$$G = \gamma * \frac{S}{l}$$

wherein $\gamma$ is conductivity (S.m$^{-1}$), S is the cross-sectional area of the material (m$^2$) and $l$ is its length (m).

The flowing electric current I (A) can then be calculated according to the formula:

$$I = U * G$$

wherein $U$ is electrical voltage (V) and G is electrical conductivity (S).

After substitution, the following formula is obtained:

$$I = U * \gamma * \frac{S}{l}$$

Because in this formula, the area S is in the numerator of the fraction, the current flow is directly proportional to this area. If this area is reduced n-fold (for example by dividing the electrode into n segments), the current required to achieve the effect of destroying the cell membrane in the food (as pulsed material) is also n-fold smaller. Because the pulse time is very short (in the order of milliseconds), sequential pulsation by electrode segments is preferably used. The total pulsation time of the material inserted between the electrodes with n segments is still very short even in the case of sequential pulsation by electrode segments, but the value of the electric current required for the pulsation is n times smaller.

The term "pulsation" or "pulsation treatment" refers to treatment of the inserted material (food) by pulsed electric field.

In a preferred embodiment, the segments of at least one contact electrode are resiliently mounted, which facilitates contact with the food to be treated and increases the contact area with the food to be treated. More preferably, the ground contact electrode segments are resiliently mounted.

The term "resiliently mounted" refers to the arrangment of the segments so that they can be tilted or compressed as needed, so that the shape of the electrode containing the segments is adapted to the inserted material (food), and thus the contact surface is increased.

Another important technical feature of the device is the AC power source. Previously proposed devices for the treatment of food by pulsed electric field use direct current. The use of alternating current leads to polarization in the material, and thus to much lower contamination of the electrodes, compared to the use of direct current.

Preferably, the device further comprises a current probe, for example, a low impedance resistor, on which the voltage drop is measured. From this information, the value of the electric current during pulsation can be derived. The current probe allows the control unit to automatically regulate the pulsation efficiency and to terminate the pulsation when a predetermined current flow rate limit is exceeded (indicating a complete or desired disruption of cell membranes in the food) or when the current flow rate does not increase any more (indicating complete cell membrane disruption).

The AC voltage source may include an isolating transformer, a rectifier module connected to the said transformer, and a capacitor bank connected to the said rectifier module and connected to the AC voltage generator. The AC voltage generator and the capacitor bank can be connected via the current probe. In this case, the device is supplied from the mains by via the isolating transformer, which reduces the supply voltage and further protects the mains supply from interference. In the rectifier module, the supply voltage is then rectified so that it can be used to charge the capacitor bank. The capacitor bank consists of a plurality of capacitors connected in parallel. They collect electric charge, which is then used for pulsing.

The AC voltage generator can be an h-bridge, which switches plus and minus very quickly (e.g. with a frequency of 10 kHz). At the beginning, the h-bridge is closed and there is therefore zero voltage in the connected circuit. The control module sends a signal whose length corresponds to the length of the pulse, and then the h-bridge starts transmitting alternating (pulsating) voltage and continues to do so during the time of receiving the signal. The h-bridge is fully controlled by the control unit.

The control unit can be a programmable microcontroller, which receives an analog signal from the current probe and controls the AC voltage generator, i.e. the beginning and the end of each pulse. Furthermore, the control unit controls the supply of voltage to the electrode segments. For this purpose, the device may comprise a relay field in which the control unit switches the relays, thereby determining the segment of the contact electrode to which the pulse is sent. The relay field consists of a number of relays corresponding to the number of segments into which the pulsation electrodes are divided. Switching on a certain relay determines the corresponding segment of the pulsation electrode to which the voltage is applied.

The control unit may preferably be configured to automatically control the end of the pulsation in the individual segments. At the first pulse in each of the segments, the value of the current flow is recorded in the memory of the control unit. In the following pulses, the increase of the value of the current flow is monitored. When the current flow value rises above a pre-defined limit in one of the segments, the pulsation in that segment is terminated because the cell membranes in the food processed in that segment are already disrupted to a predefined extent. The pulsation is also terminated when the current flow through the segment no longer increases for a pre-defined period of time.

Thus, in some embodiments, the device of the present invention includes an isolating transformer, a rectifier module, a capacitor bank, a current probe, an AC voltage generator, a control unit, a relay field, and contact (pulsation) electrodes divided into a plurality of segments. The invention further relates to a method for treating (processing) food, in particular fruits and vegetables, in which the food to be treated (processed) is inserted between the contact electrodes, which electrodes are divided into segments, and then alternating voltage pulses are applied from the AC voltage source to the individual electrode segments, said voltage pulses passing through the food and causing the discruption of cell membranes in the food. Preferably, the pulse voltage is applied to one segment of the contact electrodes at a time.

The term "food treatment" (food processing) means the disruption of the cell membranes in the food, leading to a change in the texture of the food and to an increase in its digestibility, without heating.

Brief description of Drawings

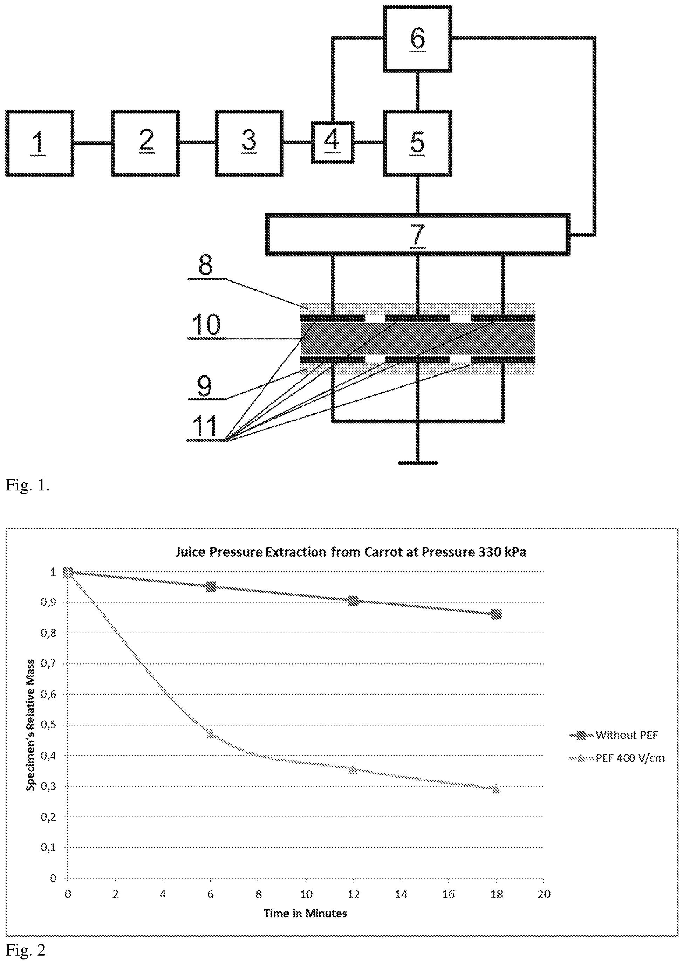

The invention is further described with the aid of Figure 1, which represents a block diagram of a kitchen electric pulse processor with divided selective automatic process control.

Fig. 2 is a graph showing the effect of pulsation treatment (PEF 400 V/cm) of carrots (i.e., disruption of cell membranes in carrots) on the amount of squeezable juice, compared to carrots not subjected to pulsation (without PEF).

## Examples of carrying out the Invention

### Example 1: Device construction

The device according to this example is shown in Figure 1. The food 10 to be treated (processed) is inserted between the working contact (pulsation) electrode 8 and the ground contact (pulsation) electrode 9, which are divided into the same number of segments 11. The food 10 is inserted between the electrode segments 11 so that the segments 11 are in contact with as large an area of the food as possible. This is ensured by the resilient mounting of the segments 11 of the grounding contact electrode 9 and by pressing the two electrodes 8 and 9 towards each other after the food 10 is inserted.

The supply voltage of the kitchen electric pulse processor is from a common electrical network (mains). This voltage is first reduced in an isolating transformer 1, while the isolating transformer 1 also protects the electrical network from interference caused by the device. The voltage coming from the isolating transformer 1 is then rectified by a rectifying module 2 so that it can be used to charge a battery 3 of capacitors. Between the battery 3 of capacitors and a generator 5 of alternating voltage, a current probe 4 is placed, by means of which the magnitude of the current flow is measured. The alternating voltage generator 5 converts the direct current voltage from the battery 3 of the capacitors back to the alternating voltage, which is then used for the pulsation. A control unit 6 controls the AC voltage generator 5. Based on the programmed algorithm and the signal received from the current probe 4, the control unit 6 determines the start and the end of the pulsation, and further controls the +/- switching and switches the current flow via the relay field 7 to the individual segments 11 of the working contact electrode 8.

### Example 2: Food processing (carrots)

To illustrate the improvement in food digestibility, an improvement in extraction of juice from pulsation-treated carrots compared to non-treated carrots can be demonstrated, as shown in Figure 2.

Cylindrical carrot samples with a diameter of 5.8 mm and a height of 5 mm were prepared. These samples were inserted between the electrodes and treated with an electric pulse with a maximum alternating voltage of 200 V (corresponding to an electric field strength of 400 V/cm), a frequency of 20 kHz and a length of 10 ms. The samples thus treated were removed and subjected to a juice extraction test.

It can be seen from Figure 2 that the weight of the pulsation-treated sample (with disrupted cell membranes) is reduced to less than 30% in 18 minutes of extraction, which means that 70% of the total weight has been extracted as juice. When juice is extracted from a non-treated sample under the same conditions, only about 15% by weight is obtained as juice in 18 minutes of extraction. The juice, which contains health-promoting substances (especially vitamins), is thus better usable by the body from a pulsation-treated sample than from a non-treated sample.

### Industrial applicability

The invention can be used for household-scale culinary preparation of fruits and vegetables. All health-promoting substances are preserved in the food treated in this way, especially vitamins. Furthermore, due to the disruption of cell membranes, the health-promoting substances are better absorbed in the human body. The invention can be used in practically every kitchen, both in the household scale and, for example, in restaurants.

## Claims

1. A food treatment device for treatment of food by pulsed electric field, comprising a control unit, a pulsed electric voltage source and at least two contact electrodes, **characterized in that** the pulsed electric voltage source is an alternating (AC) voltage source, **in that** each contact electrode (8, 9) is divided into a plurality of segments (11), and **in that** the control unit (6) is configured to control the alternating voltage source and to control the supply of voltage to the individual electrode segments (11).

2. The device according to claim 1, **characterized in that** the segments (11) of at least one contact electrode are resiliently mounted.

3. The device according to claim 1 or 2, **characterized in that** it further comprises a current probe (4).

4. The device according to any one of the preceding claims, **characterized in that** the alternating voltage source comprises an isolating transformer (1), a rectifier module (2) connected to the isolating transformer, a capacitor battery (3) connected to the rectifier module and connected to the alternating voltage generator (5), wherein the alternating voltage generator (5) and the capacitor bank (3) are preferably connected via the current probe (4).

5. The device according to any one of the preceding claims, **characterized in that** it comprises a device for switching the supply of voltage to the individual electrode segments (11), preferably a relay field (7).

6. A method of treating food by pulsed electric field using the device according to any one of the preceding claims, **characterized in that** the food (10) to be treated is inserted between contact electrodes (8, 9) which are divided into segments (11), and then a pulsed alternating voltage from an alternating voltage source is applied to the individual electrode segments (11), thus generating a pulsed electric field flowing through the food (10) and causing the disruption of cell membranes in the food (10).

7. The method according to claim 6, **characterized in that** the pulsed alternating voltage is applied to one segment (11) of the contact electrodes at a time.

8. The method according to claim 6 or 7, **characterized in that** the control unit (6) automatically regulates and/or automatically terminates the pulsation separately on each segment (11) of the electrodes (8, 9) when a pre-determined limit value of the current flow measured by a current probe (4) is exceeded, or when the current flow value measured by the current probe (4) does not increase any more.

9. The method according to claim 8, **characterized in that** during the first pulse the values of the current flow through each electrode segment (11) are recorded into the memory of the control unit (6), and during further pulsing the increase of the current flow value measured on each electrode segment (11) is monitored by a current probe (4), wherein when the current flow value in a segment (11) rises above a pre-defined limit or the current flow value in a segment (11) does not increase for a pre-determined period of time, the control unit (6) stops the pulsation on the electrode segment (11).

## Patentansprüche

1. Lebensmittelbehandlungsvorrichtung zur Behandlung von Lebensmitteln durch gepulstes elektrisches Feld, umfassend eine Steuereinheit, eine Quelle gepulster elektrischer Spannung und mindestens zwei Kontaktelektroden, **dadurch gekennzeichnet, dass** die Quelle gepulster elektrischer Spannung eine Wechselspannungsquelle ist, **dass** jede Kontaktelektrode (8, 9) in mehrere Segmente (11) unterteilt ist, und **dass** die Steuereinheit (6) zur Steuerung der Wechselspannungsquelle und zur Steuerung der Spannungsversorgung der einzelnen Elektrodensegmente (11).

2. Vorrichtung nach Anspruch 1, **dadurch gekennzeichnet, dass** die Segmente (11) mindestens einer Kontaktelektrode federnd gelagert sind.

3. Vorrichtung nach Anspruch 1 oder 2, **dadurch gekennzeichnet, dass** sie ferner eine Stromsonde (4) umfasst.

4. Vorrichtung nach einem der vorhergehenden Ansprüche, **dadurch gekennzeichnet, dass** die Wechselspannungsquelle einen Trenntransformator (1), ein mit dem Trenntransformator verbundenes Gleichrichtermodul (2), eine mit dem Gleichrichtermodul verbundene und mit dem Wechselspannungsgenerator (5) verbundene Kondensatorbatterie (3) umfasst, wobei der Wechselspannungsgenerator (5) und die Kondensatorbatterie (3) vorzugsweise über die Stromsonde (4) verbunden sind.

5. Vorrichtung nach einem der vorhergehenden Ansprüche, **dadurch gekennzeichnet, dass** sie eine Einrichtung zum Schalten der Spannungsversorgung der einzelnen Elektrodensegmente (11), vorzugsweise ein Relaisfeld (7), umfasst.

6. Verfahren zur Behandlung von Lebensmitteln durch gepulstes elektrisches Feld unter Verwendung der Vorrichtung nach einem der vorhergehenden Ansprüche, **dadurch gekennzeichnet, dass** das zu behandelnde Lebensmittel (10) zwischen Kontaktelektroden (8, 9) eingeführt wird, die in Segmente unterteilt sind (11) und anschließend wird an die einzelnen Elektrodensegmente (11) eine gepulste Wechselspannung aus einer Wechselspannungsquelle angelegt, wodurch ein gepulstes elektrisches Feld erzeugt wird, das durch das Lebensmittel (10) fließt und das Aufbrechen von Zellmembranen im Lebensmittel (10) verursacht.

7. Verfahren nach Anspruch 6, **dadurch gekennzeichnet, dass** die gepulste Wechselspannung jeweils an ein Segment (11) der Kontaktelektroden angelegt wird.

8. Verfahren nach Anspruch 6 oder 7, **dadurch gekennzeichnet, dass** die Steuereinheit (6) die Pulsation an jedem Segment (11) der Elektroden (8, 9) separat automatisch regelt und/oder automatisch beendet wenn ein vorgegebener Grenzwert des von einer Stromsonde (4) gemessenen Stromflusses überschritten wird oder wenn der von der Stromsonde (4) gemessene Stromflusswert nicht mehr ansteigt.

9. Verfahren nach Anspruch 8, **dadurch gekennzeichnet, dass** während des ersten Pulses die Werte des Stromflusses durch jedes Elektrodensegment (11) in den Speicher der Steuereinheit (6) eingetragen werden und während des weiteren Pulsens der Anstieg des an jedem Elektrodensegment (11) gemessenen Stromflusswertes wird durch eine Stromsonde (4) überwacht, wobei, wenn der Stromflusswert in einem Segment (11) eine vordefinierte Grenze überschreitet oder der Stromflusswert in einem Segment (11) für eine vorgegebene Zeitdauer nicht ansteigt, stoppt die Steuereinheit (6) die Pulsation am Elektrodensegment (11).

## Revendications

1. Appareil pour le traitement d'aliments par champ électrique pulsé, comprenant une unité de commande, une source de tension électrique pulsée et au moins deux électrodes de contact, **caractérisé en ce que** la source de tension électrique puisée est une source de tension alternative (AC), **en en ce que** chaque électrode de contact (8, 9) est divisée en plusieurs segments (11), et **en ce que** l'unité de commande (6) est configurée pour commander la source de tension alternative et pour commander l'alimentation en tension des segments d'électrode individuels (11).

2. Appareil selon la revendication 1, **caractérisé en ce que** les segments (11) d'au moins une électrode de contact sont montés élastiquement.

3. Appareil selon la revendication 1 ou 2, **caractérisé en ce qu'**il comprend en outre une sonde de courant (4).

4. Appareil selon l'une quelconque des revendications précédentes, **caractérisé en ce que** la source de tension alternative comprend un transformateur d'isolement (1), un module redresseur (2) relié au transformateur d'isolement, une batterie de condensateurs (3) reliée au redresseur et connectée au générateur de tension alternative (5), le générateur de tension alternative (5) et la batterie de condensateurs (3) étant de préférence connectés via la sonde de courant (4).

5. Appareil selon l'une quelconque des revendications précédentes, **caractérisé en ce qu'**il comprend un dispositif de commutation de l'alimentation en tension des segments d'électrodes individuels (11), de préférence un champ relais (7).

6. Procédé de traitement d'aliments par champ électrique pulsé utilisant l'appareil selon l'une quelconque des revendications précédentes, **caractérisé en ce que** l'aliment (10) à traiter est inséré entre des électrodes de contact (8, 9) qui sont divisées en segments ( 11), puis une tension alternative puisée provenant d'une source de tension alternative est appliquée aux segments d'électrode individuels (11), générant ainsi un champ électrique pulsé traversant l'aliment (10) et provoquant la rupture des membranes cellulaires dans l'aliment (10 ).

7. Procédé selon la revendication 6, **caractérisé en ce que** la tension alternative puisée est appliquée à un segment (11) des électrodes de contact à la fois.

8. Procédé selon la revendication 6 ou 7, **caractérisé en ce que** l'unité de commande (6) régule automatiquement et/ou arrête automatiquement la pulsation séparément sur chaque segment (11) des électrodes (8, 9) lorsqu'une limite prédéterminée de la valeur du flux de courant mesurée par une sonde de courant (4) est dépassée, ou lorsque la valeur du flux de courant mesurée par la sonde de courant (4) n'augmente plus.

9. Procédé selon la revendication 8, **caractérisé en ce que** pendant la première impulsion les valeurs du flux de courant à travers chaque segment d'électrode (11) sont enregistrées dans la mémoire de l'unité de commande (6), et pendant les impulsions suivantes l'augmentation de la valeur du flux de courant mesurée sur chaque segment d'électrode (11) est surveillée par une sonde de courant (4), où lorsque la valeur du flux de courant dans un segment (11) dépasse une limite prédéterminée ou la valeur du flux de courant dans un segment (11) n'augmente pas pendant une période de temps prédéterminée, l'unité de commande (6) arrête la pulsation sur ce segment d'électrode (11).

Fig. 1.

Fig. 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6746613 B2
- EP 2543254 A1
- WO 2006121397 A
- WO 2011139144 A1
- US 9565869 B2